# EUROPEAN PATENT APPLICATION

(11) **EP 2 281 824 A1**
(43) Date of publication of application: **09.02.2011**
(21) Application number: 09382139.5
(22) Date of filing: 07.08.2009
(51) Int. Cl.: C07D 405/06, C07D 405/14, C07D 413/14, A61K 31/4178, A61K 31/422, A61K 31/4439, A61K 31/506, A61P 25/00

(54) **Furan-imidazolone derivatives, for the treatment of cognitive, neurodegenerative or neuronal diseases or disorders**

(71) Applicant: NOSCIRA, S.A., 28760 Tres Cantos, Madrid (ES)
(72) Inventor: Medina Padilla, Miguel, E-28760 Tres Cantos -MADRID (ES); Sànchez-Quesada, Jorge, E-28760 Tres Cantos -MADRID (ES); Herrero Santos, Susana, E-28760 Tres Cantos -MADRID (ES); Garcià Palomero, Esther, E-28760 Tres Cantos -MADRID (ES)
(74) Representative: ABG Patentes, S.L.

(57) **Abstract**

The present invention is related to a family of furan-imidazolone derivatives of formula (I), and to their use in the treatment of cognitive, neurodegenerative or neuronal diseases or disorders, such as Alzheimer's disease or Parkinson's Disease. The present invention also relates to pharmaceutical compositions comprising the same.

## Description

### FIELD OF THE INVENTION

The present invention is related to a new family of furan-imidazolone derivatives, and to their use in the treatment of cognitive, neurodegenerative or neuronal diseases or disorders, such as Alzheimer's disease or Parkinson's Disease. The present invention also relates to pharmaceutical compositions comprising the same.

### BACKGROUND OF THE INVENTION

### BETA AMYLOID

β-Amyloid peptides (Aβ) are generated in neuronal secretory vesicles by proteolytic cleavage of the type I transmembrane amyloid precursor protein (APP) by aspartyl-proteases, called β-secretase and γ-secretase, that cleave at the N terminus and variant C-termini of Aβ within APP, respectively, resulting in Aβ of 40 or 42 amino acids (Aβ40 and Aβ42, respectively) [Hook V. et al. (2008). Biological Chemistry 389 (8): 993-1006]. As an example of their effects, both peptides have been believed to initiate aggregation and later deposition in the neuritic plaques characteristic for Alzheimer's Disease (AD) [Findeis et al. (2007). Alzheimer's disease Pharmacol. Therapeut 116: 266-286].

The literature on Aβ biology is replete with a variety of different mechanisms of action, some of which may relate to varying structural states of the peptide. In primary neuronal cultures, Aβ oligomers and Aβ-derived diffusible ligands (ADDLs) can bind avidly to neuronal membranes and induce rapid cell death through the mitochondrial apoptotic pathway. In contrast, Aβ fibrils appear to induce a more chronic form of neuritic dystrophy and neuronal cell death. Rapid toxic effects of Aβ have been associated with a pro-oxidant effect of the peptide and may be mediated in part through RAGE (Receptor for Advanced Glycation End products). Aβ can also induce apoptosis through activation of caspases and calpain. Another mechanism of toxicity may involve aberrant activation of cell cycle reentry in neurons, which has been observed in Aβ-treated neuronal cultures and in AD. Little is known about the factors that regulate the generation of toxic Aβ aggregates in the aging brain, although recent studies suggest potential roles for insulinlinsulin-like growth factor-1 signalling and calcium homeostasis. Another class of signalling pathways activated by Aβ is involved in the microglial inflammatory response. Amyloid deposits are closely associated with activation of microglia in AD and in APP transgenic mice [for a review: Yankner B.A. and Lu T. (2009). Journal of Biological Chemistry 284 (8): 4755-4759].

The presence of Aβ deposits in the brain has been related to numerous diseases, such as Alzheimer's disease [Iversen et al. (1995). Biochem J 311 (Pt 1):1-16; Sisodia S.S. (1999). J Clin Invest 104:1169-1170; Selkoe D.J., (2001). Physiol Rev 81:741-766; Gandy et al. (2003). Alzheimer Dis Assoc Disord 17:259-266], memory loss, attention deficit symptoms associated with Alzheimer's disease, diffuse Lewy body type Alzheimer's disease [Gomperts S.N. et al. (2008). Neurology, 16;71(12): 903-10], mild cognitive impairment [Frankfort S.V. et al. (2008). Curr Clin Pharmacol. 3(2): 123-31], Hereditary Cerebral Haemorrhage with Amyloidosis of the Dutch-Type [Maat-Schieman M. et al. (2005). Neuropathology, 25(4): 288-97], β-amyloid angiopathy and cerebral bleeding such as cerebral bleeding due to solitary cerebral amyloid angiopathy [Pezzini A. and Padovani A. (2008). Neurol Sci. 29 Suppl 2: S260-3], prion infections [Lupi O. and Peryassu M.A. (2007). Prion 1(4):223-7], type II diabetes [Götz J., Ittner L.M. and Lim Y.A. (2009).Cell Mol Life Sci. 66(8):1321-5], degenerative dementias, including dementias of mixed vascular and degenerative origin [Eikelenboom P. et al. (2008). Neurodegener Dis. 5(3-4):190-3], frontotemporal dementia [Rosso S.M. and van Swieten J.C. (2002). Curr Opin Neurol. 15(4): 423-8], pre-senile dementia, senile dementia [Tian J. et al. (2004). Panminerva Med. 46(4): 253-64], AIDS associated dementia [Everall I.P. et al (2005). Neurotox Res. 8(1-2): 51-61], parkinsonian disorders such as Parkinson's disease (PD), subacute sclerosing panencephalitic parkinsonism, postencephalitic parkinsonism, pugilistic encephalitis, guam parkinsonism-dementia complex, Pick's disease, multiple system atrophy (MSA), progressive supranuclear palsy (PSP), and corticobasal degeneration (CBD) [Constantinescu R. et al. (2008). Parkinsonism Relat Disord. 15(3): 205-12], Down syndrome [Lott I.T. et al. (2006). Curr Alzheimer Res. 3(5): 521-8], Lewy body disease [Hanson J.C. and Lippa C.F. (2009). Int. Rev. Neurobiol. 84:215-28], Huntington's Disease [Mena M.A. et al. (2009). Prion. 3(1): 5-11], amyotrophic lateral sclerosis [Harrison R.S. et al. (2007). Rev Physiol Biochem Pharmacol. 159: 1-77], multiple sclerosis [Mattsson N. et al. (2009). Mult Scler. 15(4): 448-54] and neurotraumatic diseases such as acute stroke [Smith E.E. and Greenberg S.M. (2009). Stroke. 40(7): 2601-6], epilepsy [Palop J.J. and Mucke L. (2009). Arch Neurol. 66(4): 435-40], mood disorders such as depression, schizophrenia and bipolar disorders [Lavretsky H. et al. (2009). Am. J. Geriatr. Psychiatry. 17(6): 493-502], promotion of functional recovery post stroke, ischaemia [Guglielmotto M. et al. (2009). J Neurochem. 108(4): 1045-56], brain injury, especially traumatic brain injury [Chen X.H. et al. (2004). Am. J. Pathol. 165(2):357-71], inflammation and chronic inflammatory diseases [Salminen A. et al. (2009). Prog Neurobiol. 87(3):181-94].

The importance of this therapeutic potential of inhibiting the deposition of β-amyloid peptides has motivated many researchers to isolate and characterize secretase enzymes and try to identify potential inhibitors thereof (see, e.g., WO01/23533 A2, EP0855444, WO00/17369, WO00/58479, WO00/47618, WO00/77030, WO01/00665, WO01/00663, WO01/29563, WO02/25276, US5,942,400, US6,245,884, US6,221,667, US6,211,235, WO02/02505, WO02/02506, WO02/02512, WO02/02518, WO02/02520, WO02/14264, WO05/058311, WO05/097767, WO06/041404, WO06/041405, WO06/0065204, WO06/0065277, US2006287294, WO06/138265, US20050282826, US20050282825, WO06/138217, WO06/138230, WO06/138264, WO06/138265, WO06/138266, WO06/099379, WO06/076284, US20070004786, US20070004730, WO07/011833, WO07/011810, US20070099875, US20070099898, WO07/049532, WO07/149033). These documents describe the development of drugs as a treatment and/or prophylaxis of Aβ-related pathologies such as Downs syndrome and β-amyloid angiopathy, such as but not limited to cerebral amyloid angiopathy, hereditary cerebral hemorraghe, disorders associated with cognitive impairment, such as but not limited to MCI ("mild cognitive impairment"), Alzheimer's disease, memory loss, attention deficit symptoms associated with Alzheimer's disease, neurodegeneration associated with diseases such as Alzheimer's disease or dementia including dementia of mixed vascular and degenerative origin, pre-senile dementia, senile dementia and dementia associated with Parkinson's disease, progressive supranuclear palsy or cortical basal degeneration.

Therefore, due to the involvement of β-amyloid in these diseases, there is a need to develop therapeutic agents that effectively reduce β-amyloid deposition.

Particularly, Alzheimer's disease (AD) is a neurodegenerative disorder characterized by the presence of β-amyloid protein (Aβ) deposits in the core of neuritic plaques and neurofibrillary tangles (NFTs) in the brain of AD patients. Abnormal accumulation of neurotoxic Aβ peptides is a significant factor in the development of AD, and thought to be the likely cause of memory and cognitive loss in this disease condition [Iversen et al. (1995). Biochem J 311 (Pt 1):1-16; Sisodia S.S. (1999). J Clin Invest 104:1169-1170; Selkoe D.J., (2001). Physiol Rev 81:741-766; Gandy et al. (2003). Alzheimer Dis Assoc Disord 17:259-266]. Neuropathological examination of brains from AD patients reveals that the accumulation of secreted Aβ peptides in extracellular amyloid plaques is involved in neuronal loss in brain regions (hippocampus and cortex) responsible for memory and cognitive functions. Clearly, strategies to reduce Aβ production will facilitate development of therapeutic agents for the treatment of AD [Hook V. et al. (2008). Biological Chemistry 389 (8): 993-1006].

### BACE

BACE (β-site amyloid precursor protein cleaving enzyme) is a transmembrane aspartyl protease of type I topology with β-secretase activity, in which the N-terminus and catalytic site reside on the luminal or extracellular side of the membrane. In fact, BACE is the major β-secretase responsible for Aβ generation in the brain. BACE catalyses the initial step in the amyloidogenic metabolism of the large transmembrane amyloid precursor protein (APP), releasing a soluble APPβ (sAPPβ) ectodomain by specific hydrolysis at the β-site (Met671-Asp672) and simultaneously generating a membrane-bound, C-terminal fragment consisting of 99 amino acids (CTF99). The latter is then further processed by the γ-secretase enzyme complex which, in turn, generates the APP intracellular domain and releases the 39-42-amino-acid amyloid β-peptide (Aβ). The identification of the Aβ peptide as the main constituent of the extracellular plaques which characterize AD led to the formulation of the 'amyloid cascade' hypothesis of AD. Interruption of this metabolic cascade at one of several sites could potentially reduce the amyloid burden, and slow or even reverse the devastating consequences of the disease. β-secretase is particularly attractive in this context, as it catalyses the first and rate-limiting step in the pathway. Moreover, BACE-1 is the major β-secretase responsible for Aβ generation in the brain. Thus, β-secretase inhibition aimed at blocking what is believed to be excessive activity or altered control of BACE-1 in AD is a key target for therapeutic intervention [Vassar (2004). J. Mol. Neurosci. 23, 105-114; Hunt C.E. and Turner A.J. (2009). FEBS Journal 276: 1845-1859].

BACE-1 mRNA and enzyme activity levels are highest in the brain, with lower expression in peripheral tissues, consistent with its role as an APP β-secretase. In the brain, BACE-1 is largely expressed by neurons, with seemingly little produced by glial cells. However, in animal models of chronic gliosis and in brains of AD patients, BACE-1 expression can be detected in reactive astrocytes, suggesting that astrocyte activation may play a role in the development of AD (for a review, Rossner S. et al. (2005). J Neurochem 92, 226-234). Hence, targeting astrocyte activation could be a viable strategy in the treatment of AD for this and other reasons [Hunt C.E. and Turner A.J. (2009). FEBS Journal 276: 1845-1859].

A variety of physiological stressors and signalling pathways have been found to regulate BACE-1 and may be a factor in the reported increased BACE-1 protein levels and enzyme activity in AD brains [Harada H. et al. (2006). Neurosci Res 54: 24-29; Li R. et al. (2004). Proc Natl Acad Sci USA 101: 3632-3637; Stockley J.H., Ravid R. and O'Neill C. (2007). FEBS Lett 580: 6550-6552.], although BACE-1 transcript levels generally appear unchanged in AD brains. Hypoxia and ischaemia are important risk factors for AD. Furthermore, oxidative stress can stimulate BACE-1 expression in cells through the c-jun N-terminal kinase pathway in a mechanism which requires the presence of presenilin [Tamagno E. et al. (2008). J Neurochem 104: 683-695]. The lipid peroxidation product 4-hydroxynonenal also upregulates BACE-1 expression through the stress activated protein kinase pathway [Tamagno E. et al. (2005). J Neurochem 92, 628-636]. The activation of cyclin-dependent kinase 5 also leads to increased levels of BACE-1 mRNA and protein *in vivo* and *in vitro,* and the BACE-1 promoter contains a cyclindependent kinase 5-responsive region [Wen Y. et al. (2008). Neuron 57: 680-690]. Other stressors that can cause the activation of BACE-1 expression include traumatic brain injury and infection of neuronal cells with herpes simplex virus 1. Additional studies have shown that cellular energy deprivation (glucose deprivation in cell culture) produces a post-transcriptional increase in BACE-1 levels which is indeed mediated through increased elF2a phosphorylation [Lammich S, et al. (2004). EMBO Rep 5: 620-625]. Thus, a common mechanism by which stress (e.g. hypoxia/ischaemia, viral infection, etc.) can influence BACE-1 levels may be through the regulation of translation initiation at the level of eIF2a. BACE-1 protein stability can also be influenced by the lysosomal and proteasomal pathways and through its lysine acetylation status (for a review: Hunt C.E. and Turner A.J. (2009). FEBS Journal 276: 1845-1859].

In summary, BACE inhibitors are described to be useful to treat and/or prevent cognitive, neurodegenerative or neuronal diseases or disorder, selected from Alzheimer's disease, memory loss, attention deficit symptoms associated with Alzheimer's disease, diffuse Lewy body type Alzheimer's disease, mild cognitive impairment, Hereditary Cerebral Haemorrhage with Amyloidosis of the Dutch-Type, β-amyloid angiopathy and cerebral bleeding such as cerebral bleeding due to solitary cerebral amyloid angiopathy, prion infections, degenerative dementias, including dementias of mixed vascular and degenerative origin, frontotemporal dementia, pre-senile dementia, senile dementia, AIDS associated dementia, parkinsonian disorders such as Parkinson's disease (PD), subacute sclerosing panencephalitic parkinsonism, postencephalitic parkinsonism, pugilistic encephalitis, guam parkinsonism-dementia complex, Pick's disease, multiple system atrophy (MSA), progressive supranuclear palsy (PSP), and corticobasal degeneration (CBD), Down syndrome, Lewy body disease, Huntington's Disease, amyotrophic lateral sclerosis, multiple sclerosis and neurotraumatic diseases such as acute stroke, epilepsy, promotion of functional recovery post stroke, ischaemia, brain injury, especially traumatic brain injury and neuroinflammation.

To date, the screening of extensive libraries for non-peptide-based BACE-1 inhibitors has resulted in the discovery of relatively few, generally low-affinity, compounds, indicating that this is not an easy protein target to inhibit effectively *in vivo.* Therefore, due to the interest of BACE as a pharmaceutical target, there is a need to develop therapeutic agents that are effective BACE inhibitors.

### CHEMISTRY

In Schuisky P. et al, Heterocycles, 1998, 48(7), 1431-1444 the synthesis of 2-amino-3-methyl-5-(5-methyl-furan-2-ylmethylene)-3,5-dihydro-imidazol-4-one is described. The synthesis of 2-amino-5-(furan-2-ylmethylene)-1,5-dihydro-imidazol-4-one is described in Mukerjee, AK et al, Heterocycles, 1991, 32(7), 1317-1325. In neither document further indication of a therapeutic use is given. WO 2009/020580 discloses aryl-methylidene substituted heterobicyclic compounds. These derivatives have been found useful as gamma-secretase modulators. FR 2919608 refers to imidazolone derivatives substituted by (Z)-methylidene groups directly attached to phenyl and pyridine substituents. The use of said compounds for the treatment of neurodegenerative diseases is disclosed, due to their ability to inhibit DYRK1A Kinase. WO2009/045314 discloses 5-membered, nitrogen-containing heterocyclic compounds. They are described as modulators of gamma secretase, although no experimental results are provided to proof this activity, but only some chemical data for some particular compounds are given.

### SUMMARY OF THE INVENTION

The present invention is related to a new family of furan-imidazolone derivatives of formula (I). They have shown an interesting activity as BACE inhibitors in *in vitro* assays. Further, some of the compounds have been tested for their capacity of inhibiting β-amyloid secretion. As detailed above, the inhibition of BACE enzyme activity and β-amyloid secretion are a good therapeutic target for the treatment of a number of diseases and conditions. Thus, taking into account that BACE and β-amyloid deposition are known to be involved in a variety of cognitive, neurodegenerative or neuronal diseases or disorders, and that their inhibition is known to help to prevent and treat these diseases, the compounds of formula (I) are useful for the prevention and/or treatment of cognitive, neurodegenerative or neuronal diseases or disorders.

Therefore, in a first aspect, the present invention is related to a compound of formula (I), or a tautomer, salt, solvate or prodrug thereof (compounds of the invention) 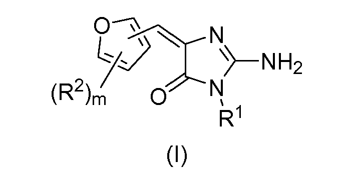 wherein
R¹ is selected from hydrogen, substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl and substituted or unsubstituted heteroaryl;
each R² is independently selected from hydrogen, substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl, substituted or unsubstituted heteroaryl, trifluoromethyl, cyano, -N(Rₐ)(R_{b}), -ORₐ, -SRₐ, -C(O)Rₐ, -C(O)ORₐ, -C(O)N(Rₐ)(R_{b}), wherein each Rₐ and R_{b} are independently selected from hydrogen and alkyl, substituted or unsubstituted alkenyl, substituted or unsubstituted alkynyl;
m is an integer selected from 1, 2 and 3;
with the proviso that:
- 2-amino-5-(furan-2-ylmethylene)-1,5-dihydro-imidazol-4-one, and
- 2-amino-3-methyl-5-(5-methyl-furan-2-ylmethylene)-3,5-dihydro-imidazol-4-one
are not included in formula (I).

Another aspect of the present invention is a compound of formula (I), or a tautomer, salt, solvate or prodrug thereof, for use as a medicament.

A further aspect of the present invention refers to a compound of formula (I), or a tautomer, salt, solvate or prodrug thereof, for use as a medicament for the treatment and/or prophylaxis of a cognitive, neurodegenerative or neuronal disease or disorder.

The present invention is further related to a pharmaceutical composition comprising at least one of the compounds of formula (I), or tautomers, salts, solvates or prodrugs thereof, and at least one pharmaceutically acceptable carrier, adjuvant and/or vehicle.

A further aspect of the present invention is a method for the preparation of a compound of formula (I) as defined above, or a tautomer, salt, solvate or prodrug thereof.

In a further aspect, the present invention is related to a method of treating and/or preventing a cognitive, neurodegenerative or neuronal disease or disorder, which method comprises administering to a patient in need of such a treatment a therapeutically effective amount of at least one compound of formula (I) or a pharmaceutical composition thereof.

In another aspect, the present invention is related to the use of a compound of formula (I), or tautomers, salts, solvates or prodrugs thereof, as a reagent in an in vivo biological assay, wherein BACE activity and/or beta-amyloid secretion is needed to be inhibited.

### DETAILED DESCRIPTION OF THE INVENTION

In the above definition of compounds of formula (I) the following terms have the meaning indicated:
The term "alkyl" refers to a linear or branched hydrocarbon chain radical consisting of carbon and hydrogen atoms, containing no instauration, having one to twelve, or one to eight, or one to six, or one to three carbon atoms, and which is attached to the rest of the molecule by a single bond. Examples of alkyl groups include, but are not limited to alkyl groups such as methyl, ethyl, n-propyl, isopropyl, 3-methyl-1-pentyl, 4-methyl-1-pentyl, 3,3-dimethyl-1-butyl, t-butyl, pentyl, isopentyl, and hexyl.

The terms "alkenyl" and "alkynyl" refer to linear or branched hydrocarbon chain radical having one or more carbon-carbon double bonds or one or more carbon-carbon triple bonds, respectively, and having from two to twelve carbon atoms, and which are attached to the rest of the molecule by a single bond. In an embodiment of the invention the alkenyl or the alkynyl has two to eight, two to six, two or three carbon atoms. The double bond of an alkenyl or the triple bond of an alkynyl group can be unconjugated or conjugated to another unsaturated group. Suitable alkenyl groups include, but are not limited to alkenyl groups such as vinyl, allyl, butenyl, butadienyl, or pentadienyl. Suitable alkynyl groups include, but are not limited to alkynyl groups such as -CCH, -CH₂CCH, -CCCH₃, -CH₂CCCH₃.

The term "aryl" refers to an aromatic group having between 6 to 18, or between 6 to 10, or 6, 7 or 8 carbon atoms, comprising 1, 2 or 3 aromatic nuclei, optionally fused, including the following non-limiting examples: phenyl, naphthyl, diphenyl, indehyl, phenantryl.

"Heteroaryl" refers to a stable 3- to 15-membered aromatic ring radical, which consists of carbon atoms and from one to five heteroatoms selected from the group consisting of nitrogen, oxygen and sulphur. In an embodiment of the invention the heteroaryl group is 3- to 10-, or a 5- or 6-membered aromatic ring radical. For the purposes of this invention, the heteroaryl can be monocyclic, bicyclic or tricyclic ring system, which can include systems of fused rings; and the nitrogen, carbon or sulphur atoms in the heteroaryl radical may be optionally oxidized; or the nitrogen atom may be optionally quaternized. Example of such heteroaryl include, but are not limited to, benzimidazole, benzothiazole, furan, pyrrole, thiophene, pyridine, pyrimidine, isothiazole, isoxazol, imidazole, indole, purine, quinoline, thiadiazole. Preferably, heteroaryl refers to pyridine, pyrimidine or isoxazol.

The term "arylalkyl" relates to an alkyl group substituted with an aryl group as defined above. Examples of such groups include, but are not limited to, benzyl, phenylethyl, phenylpropyl, naphthylmethyl,etc. In an embodiment of the invention the term "arylalkyl" refers to a group having between 7 and 16 carbon atoms ("C₇₋₁₆ arylalkyl"). In a further embodiment, the arylalkyl group comprises 7 to 11 carbon atoms.

The term "heteroarylalkyl" relates to an alkyl group substituted with a heteroaryl group as defined above. Preferably, the term "heteroarylalkyl" refers to a group having between 4 and 16 carbon atoms ("C₄₋₁₆ heteroarylalkyl").

The term "halogen" refers to chloro, bromo, iodo or fluoro.

References herein to substituted groups in the compounds of the present invention refer to the specified moiety that may be substituted at one or more available positions by one or more suitable groups, e. g., halogen; cyano; nitro; azido; C₁₋₆ alkyl; C₁₋₆ alkenyl; C₁₋₆ alkynyl; C₆₋₁₀ aryl; 5- or 6-membered heteroaryl, C₇₋₁₁ arylalkyl, 5- or 6-membered heteroaryl, (C₁₋₆)alkyl, -OR, -SR, -SOR, -SO₂R -NRR', -C(O)R, -CO₂R or - C(O)N(R)(R'), wherein R and R' are each independently selected from hydrogen, C₁₋₆ alkyl, C₆₋₁₀ aryl, 5- or 6-membered heteroaryl, C₇₋₁₁ arylalkyl and heteroaryl(C₁₋₆)alkyl. Unless otherwise indicated, an optionally substituted group may have a substituent at each substitutable position of the group, and each substitution is independent of the other.

### Compounds of formula (I)

According to a particular embodiment, the present invention is directed to a compound of formula (la) 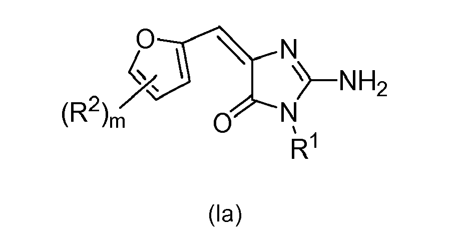 wherein R¹, R² and m are as previously defined, or a tautomer, salt, solvate or prodrug thereof.

In a particular embodiment, m is selected from 1 or 2. In an embodiment of the invention m is 1.

When m is 2 or 3, the R² groups may be the same or different.

In a further particular embodiment, R² is hydrogen, the furane unit being unsubstituted.According to another particular embodiment, the present invention is directed to a compound of formula (Ib) 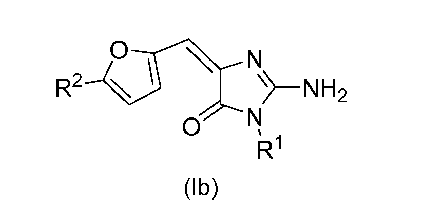 wherein R¹ and R² are as previously defined, or a tautomer, salt, solvate or prodrug thereof.

Different isomers may arise depending on the double bond geometry of the furan-2-ylmethylene substituent (e.g. Z, E). The pure single isomers Z and E, and mixtures thereof in different percentages, fall within the scope of the present invention.

According to an embodiment of the invention, the double bond geometry is (E).

I n a particular embodiment, R¹ is selected from hydrogen, substituted or unsubstituted C₁₋₃ alkyl,. substituted or unsubstituted arylmethyl, substituted or unsubstituted heteroarylmethyl, substituted or unsubstituted phenyl and substituted or unsubstituted heteroaryl. Preferably R¹ is selected from hydrogen, methyl, substituted or unsubstituted benzyl, substituted or unsubstituted heteroarylmethyl, substituted or unsubstituted phenyl and substituted or unsubstituted methyloxyethyl.

In one embodiment, each R² is independently selected from substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl, substituted or unsubstituted heteroaryl, trifluoromethyl, cyano, -N(Rₐ)(R_{b}), -ORₐ, -SRₐ, -C(O)Rₐ, -C(O)ORₐ, - C(O)N(Rₐ)(R_{b}), wherein each Rₐ and R_{b} are independently selected from hydrogen and alkyl, substituted or unsubstituted alkenyl, substituted or unsubstituted alkynyl.

In another particular embodiment R² is selected from hydrogen and substituted or unsubstituted alkyl. In yet a further embodiment R² is selected from hydrogen and C₁₋₃ alkyl, such as methyl. In a further particular embodiment, R² is a substituted C₁₋₃ alkyl, preferably substituted by C₁₋₃ alkyloxy.

According to a particular embodiment, each R² is selected from substituted or unsubstituted arylalkyl, substituted or unsubstituted heteroarylalkyl, substituted or unsubstituted aryl, substituted or unsubstituted heteroaryl. In yet a further embodiment, R² is selected from substituted or unsubstituted aryl, substituted or unsubstituted heteroaryl.

According to another particular embodiment, R² is selected from C₁₋₃ alkyl, substituted or unsubstituted phenyl and susbstituted or unsubstituted 5- or 6-membered heteroaryl ring, such as a susbstituted or unsubstituted 5- or 6-membered N-containing heteroaryl ring. In yet a further embodiment R² is selected from methyl, substituted or unsubstituted phenyl, substituted or unsubstituted pyridine, substituted or unsubstituted pyrimidine or substituted or unsubstituted isoxazol. In yet a further embodiment, R² is a phenyl, pyridine, pyrimide or an isoxazol ring substituted with 1, 2 or 3 groups selected from C₁₋₃ alkyl, preferably methyl; -O(C₁₋₃ alkyl), preferably methoxy; -CO₂(C₁₋₃ alkyl), preferably methoxycarbonyl; halogen, nitro, cyano or [1,3]dioxolane. In another embodiment, R² is selected from hydrogen, methyl, or a group selected from the following:

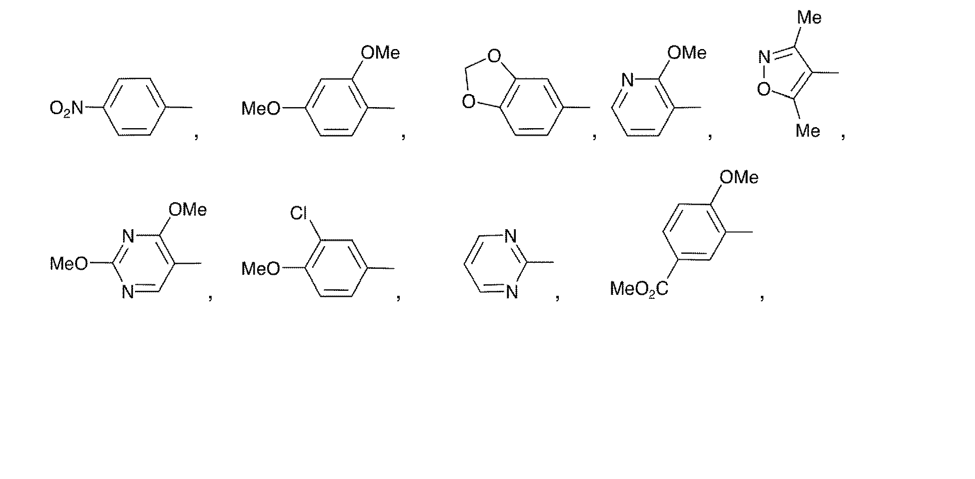

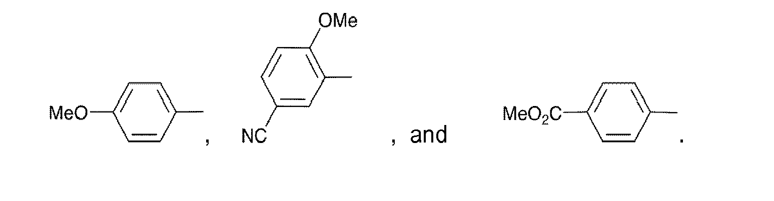

In the above detailed groups, the bond not having any detailed substituent is the position at which the groups bond directly to the furane within the compounds of formula (I).

In a further embodiment of the invention, R² is selected from hydrogen, C₁₋₄ alkyl, and a group of formula (A), (B) or (C) 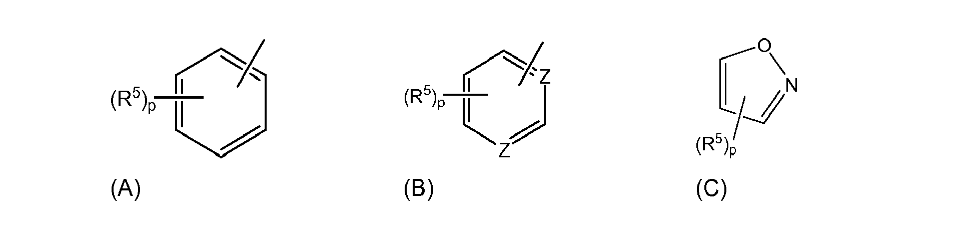 wherein
p is an integer selected from 0,1,2 and 3; and each R⁵ is independently selected from C₁₋₃ alkyl, -O( C₁₋₃ alkyl), nitro, halogen, cyano and C(=O)ORc, wherein Rc is selected from hydrogen and C₁₋₆ alkyl.

According to another embodiment of the present invention, the compound of formula (I) is selected from:

| | |
|---|---|
| **Compound 1** | 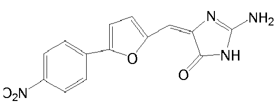 |
| **Compound 2** | 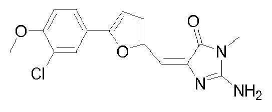 |
| **Compound 3** | 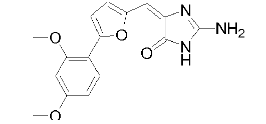 |
| **Compound 4** | 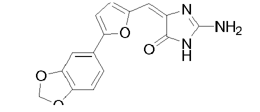 |
| **Compound 5** | 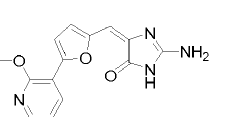 |
| **Compound 6** | 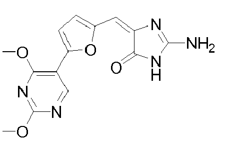 |
| **Compound 7** | 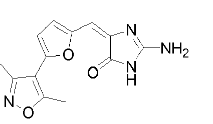 |
| **Compound 8** | 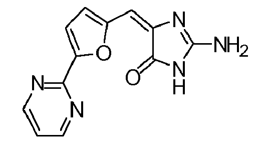 |
| **Compound 9** | 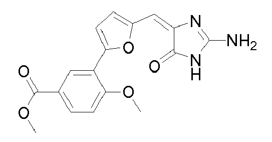 |
| **Compound 10** | 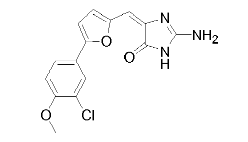 |
| **Compound 11** | 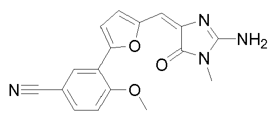 |
| **Compound 12** | 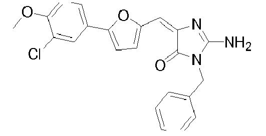 |
| **Compound 13** | 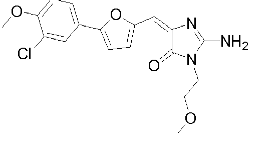 |
| **Compound 14** | 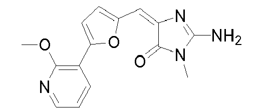 |
| **Compound 15** | 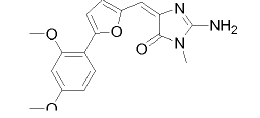 |
| **Compound 16** | 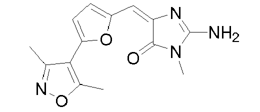 |
| **Compound 17** | 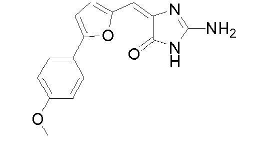 |
| **Compound 18** | 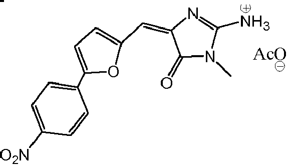 |

or a tautomer, salt, solvate or prodrug thereof.

The compounds of the invention may comprise asymmetric substituents, i.e. asymmetric substituents in R₁ and/or R₂, which may give raise to the presence of different stereoisomers (enantiomer, stereoisomers, etc). Also different isomers may arise depending on the presence of multiple bonds (e.g. Z, E). The single isomers, enantiomers or diastereoisomers and mixtures thereof fall within the scope of the present invention.

The compounds of the invention may be in the form of tautomers, in the form of salts, preferably pharmaceutically acceptable salts, in the form of solvates or in the form of prodrugs.

The term "pharmaceutically acceptable salts" refers to salts which, upon administration to the recipient are capable of providing (directly or indirectly) a compound as described herein. The preparation of salts can be carried out by methods known in the art. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

The term "prodrug" as used in this application is defined here as meaning a chemical compound having undergone a chemical derivation such as substitution or addition of a further chemical group to change (for pharmaceutical use) any of its physico-chemical properties, such as solubility or bioavailability, e.g. ester and ether derivatives of an active compound that yield the active compound per se after administration to a subject. Examples of well known methods of producing a prodrug of a given acting compound are known to those skilled in the art and can be found e.g. in Krogsgaard-Larsen et al., Textbook of Drug design and Discovery, Taylor & Francis (April 2002).

Particularly favoured prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The term "solvate" according to this invention is to be understood as meaning any form of the active compound according to the invention which has another molecule (most likely a polar solvent) attached to it via a non-covalent bonding. Examples of such solvates include hydrates and alcoholates, e.g. methanolates.

The preparation of salts, solvates and prodrugs can be carried out by methods known in the art. It will be appreciated that non-pharmaceutically acceptable salts, solvates or prodrugs also fall within the scope of the invention since those may be useful in the preparation of pharmaceutically acceptable salts, solvates or prodrugs.

The compounds of the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of the present invention may exhibit tautomerism. Tautomers are one of two or more structural isomers of a compound that exist in equilibrium and are readily converted from one isomeric form to another. Common tautomeric pairs are amine-imine, amide-imidic acid, keto-enol, lactam-lactim, etc. For example, the compounds of the present invention may be equally represented as an imino-imidazolidinone ring or as the corresponding amino-dihydroimidazolones (see Figure 1 below).

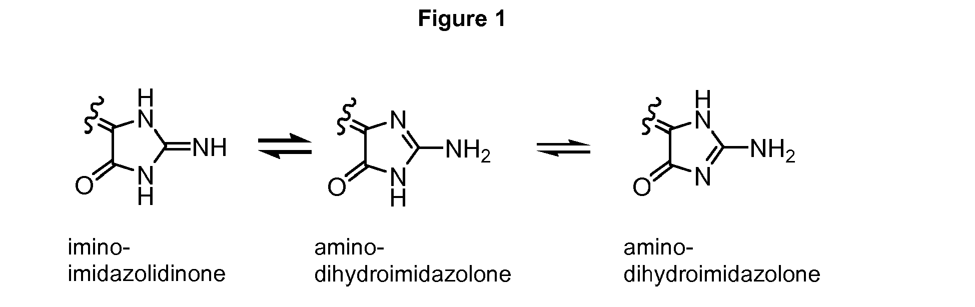

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

### Synthesis of compounds of formula (I)

The compounds of formula (I) can be prepared by condensing the corresponding heteroaryl-aldehyde of formula (II) and the imino-imidazolidinone ring of formula (III), as follows:

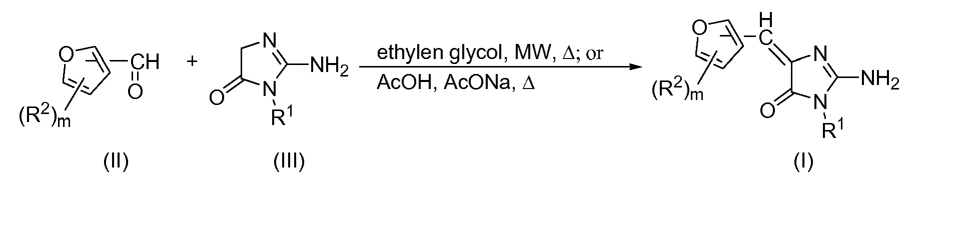

See also Heterocycles, 1998, 48, 1431-1444; Synthetic Comm. 1995, 25, 3135-3140 or Heterocycles, 1998, 48, 1575-1580.

Such heteroaryl-aldehydes of formula (II) are either commercially available or prepared by Suzuki coupling reaction of the corresponding halogen-substituted heteroaryl aldehyde of formula (IV) with R²-B(OH)₂ (Method A) or from the corresponding boronic acid of the heteroaryl aldehyde of formula (V) with R²-X, wherein X is halogen (Method B). The coupling reaction is carried out in the presence of a palladium catalyst and a base. In a preferred embodiment, Pd(PPh₃)₄ is used as catalyst in the presence of an inorganic base, such as sodium bicarbonate, and an organic solvent, preferably tetrahydrofuran.

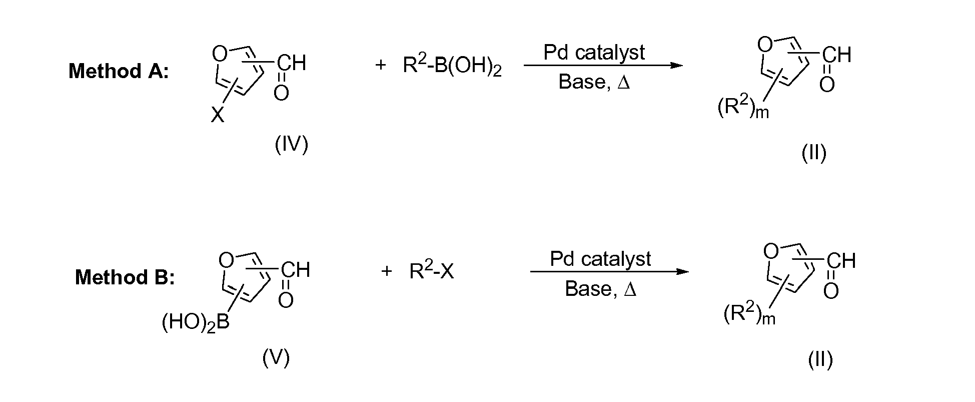

### Uses of compounds of formula (I)

Generally a "therapeutically effective amount" of the compound of the invention or a pharmaceutical composition thereof will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

The compounds used in the present invention may be used with at least one other drug to provide a combination therapy. The at least other drug may be part of the composition, or be provided as a separate composition for administration at the same time or at different time. According to one embodiment, the at least other drug is a compound useful for the treatment and/or prophylaxis of cognitive, neurodegenerative or neuronal diseases or disorders. According to another embodiment, the at least other drug is selected from the group comprising beta secretase inhibitors or modulators, muscarinic antagonists, acetylcholinesterase inhibitors, butyrilcholinesterase inhibitors, gamma secretase inhibitors or modulators, HMG-CoA reductase inhibitors, non-steroidal anti-inflammatory agents, N-methyl-D-aspartate receptor antagonists, anti-amyloid antibodies, vitamin E, nicotinic acetylcholine receptor agonists, serotonin receptor modulators, CB1 receptor inverse agonists or CB1 receptor antagonists, AMPA agonists, GABA_{A} inverse agonists, inhibitors of amyloid aggregation, glycogen synthase kinase beta inhibitors, promoters of alpha secretase activity, PDE-10 inhibitors, estrogen and cholesterol absorption inhibitors.

In a particular embodiment the present invention is directed to a compound of formula (I) as defined above, or a tautomer, salt, solvent or prodrug thereof, for use as a medicament for the treatment and/or prophylaxis of a cognitive, neurodegenerative or neuronal disease or disorder selected from chronic neurodegenerative conditions including dementias such as Alzheimer's disease, memory loss, attention deficit symptoms associated with Alzheimer's disease, diffuse Lewy body type Alzheimer's disease, mild cognitive impairment, Hereditary Cerebral Haemorrhage with Amyloidosis of the Dutch-Type, β-amyloid angiopathy and cerebral bleeding such as cerebral bleeding due to solitary cerebral amyloid angiopathy, prion infections, degenerative dementias, including dementias of mixed vascular and degenerative origin, frontotemporal dementia, pre-senile dementia, senile dementia, AIDS associated dementia, parkinsonian disorders such as Parkinson's disease (PD), subacute sclerosing panencephalitic parkinsonism, postencephalitic parkinsonism, pugilistic encephalitis, guam parkinsonism-dementia complex, Pick's disease, multiple system atrophy (MSA), progressive supranuclear palsy (PSP), and corticobasal degeneration (CBD), Down syndrome, Lewy body disease, Huntington's Disease, amyotrophic lateral sclerosis, multiple sclerosis and neurotraumatic diseases such as acute stroke, epilepsy, promotion of functional recovery post stroke, ischaemia, brain injury, especially traumatic brain injury and neuroinflammation. In preferred embodiments, the disease or disorder is selected form Alzheimer's Disease, Parkinson's Disease, amyotrophic lateral sclerosis, multiple sclerosis, acute stroke and ischaemia.

In another particular embodiment, the present invention is directed to a compound of formula (I) as defined above, or a tautomer, salt, solvent or prodrug thereof, for use as a medicament for the treatment and/or prophylaxis of a disease or condition selected from type II diabetes, mood disorders such as depression, schizophrenia and bipolar disorders, inflammation and chronic inflammatory diseases.

According to a further aspect, the present invention is directed to the use of a compound of formula (I) as defined above, or a tautomer, salt, solvent or prodrug thereof, in the preparation of a medicament for the treatment and/or profilaxis of a cognitive, neurodegenerative or neuronal disease or disorder.

### Pharmaceutical compositions

The present invention further provides pharmaceutical compositions comprising at least a compound of formula (I) of the present invention, or a tautomer, salt, solvate or prodrug thereof, and at least one pharmaceutically acceptable carrier, adjuvant, and/or vehicle, for administration to a patient.

The term "carrier, adjuvant and/or vehicle" refers to molecular entities or substances with which the active ingredient is administered. Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin.

According to one embodiment, the pharmaceutical composition may further contain a therapeutically effective amount of one or more compounds useful for the treatment and/or prophylaxis of cognitive, neurodegenerative or neuronal diseases or disorders. According to another embodiment, the pharmaceutical composition may further contain a therapeutically effective amount of one or more compounds selected from the group comprising beta secretase inhibitors or modulators, muscarinic antagonists, acetylcholinesterase inhibitors, butyrilcholinesterase inhibitors, gamma secretase inhibitors or modulators, HMG-CoA reductase inhibitors, non-steroidal anti-inflammatory agents, N-methyl-D-aspartate receptor antagonists, anti-amyloid antibodies, vitamin E, nicotinic acetylcholine receptor agonists, serotonin receptor modulators, CB1 receptor inverse agonists or CB1 receptor antagonists, AMPA agonists, GABA_{A} inverse agonists, inhibitors of amyloid aggregation, glycogen synthase kinase beta inhibitors, promoters of alpha secretase activity, PDE-10 inhibitors, estrogen and cholesterol absorption inhibitors.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form because of the convenience for the patient and the chronic character of many of the diseases to be treated. Suitable dosage forms for oral administration may be tablets or capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

In the following, the present invention is further illustrated by examples. They should in no case be interpreted as a limitation of the scope of the invention as defined in the claims.

### EXAMPLES

Compounds of formula (I) according to the present invention were prepared following the general preparation strategy detailed below. The detailed preparation of some of the compounds is described hereinafter.

All the reagents used are commercially available.

### Synthesis of aldehyde intermediates

### General procedure for the Suzuki coupling. Method A

To a solution of the halogen-substituted heteroaryl aldehyde (2.7 mmol) of formula (IV) and the corresponding boronic acid (3.2 mmol) of formula R²-B(OH)₂ (R² being as defined above) in THF (20.0 mL) a solution of sodium bicarbonate (8.0 mmol) in water (9.0 mL) was added. The reaction mixture was stirred at room temperature for 5 minutes, and a solution of Pd(PPh₃)₄ (0.2 mmol) in THF (5.0 mL) was added. The reaction mixture was refluxed at 80° C for three days, cooled to room temperature and diluted with ethyl acetate (150.0 mL) and water (50.0 mL). The aqueous layer was extracted with ethyl acetate (2 × 25.0 mL), and the combined organic layers were washed with brine, dried with anhydrous sulfate, filtered and concentrated under vacuum to afford the corresponding aldehyde of formula (II).

### General procedure for the Suzuki coupling. Method B

To a solution of the heteroaryl aldehyde boronic acid (2.7 mmol) of formula (V) and the appropriate halide (3.2 mmol) of formula R²-X (R² being as defined above) in THF (20 mL) a solution of sodium carbonate (8.0 mmol) in water (9.0 mL) was added. The reaction mixture was stirred at room temperature for 5 minutes, and a solution of Pd(PPh₃)₄ (0.2 mmol) in THF (5.0 mL) was added. The reaction mixture was refluxed at 80° C for three days until completion, cooled to room temperature and diluted with ethyl acetate (150.0 mL) and water (50.0 mL). The aqueous layer was extracted with ethyl acetate (2 × 25.0 mL), and the combined organic layers were washed with brine, dried with anhydrous sodium sulfate, filtered and concentrated under vacuum to afford the corresponding aldehyde of formula (II).

### Example 1. Synthesis of 5-(2,4-dimethoxy-phenyl)-furan-2-carbaldehyde

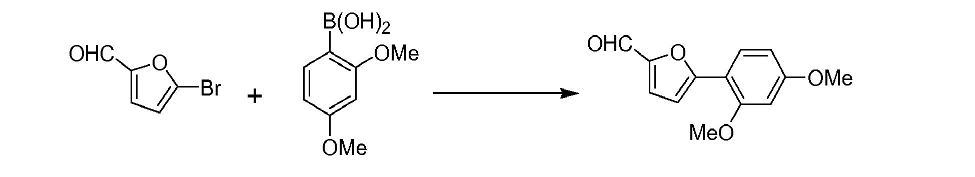

Synthesized using method A. The compound did not need any further purification. 630.0 mg; 100% yield.
MS (ES): M/z= 233 (M+H)⁺
¹H NMR (400 MHz, CDCl₃, δ ppm): 9.59 (s, 1 H), 7.98 (d, J = 8.7 Hz, 1 H), 7.31 (d, J = 3.7 Hz, 1 H), 7.00 (d, J = 3.7 Hz, 1 H), 6.60 (dd, J = 8.7, 2.3 Hz, 1 H), 6.53 (d, J = 2.3 Hz, 1 H), 3.94 (s, 3H), 3.87 (s, 3H).

### Example 2. Synthesis of 5-benzo[1,3]dioxol-5-yl-furan-2-carbaldehyde

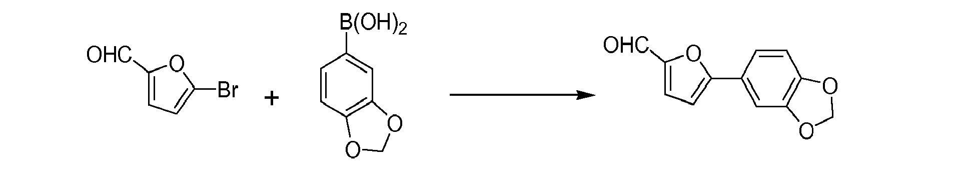

Synthesized using method A and purified by flash chromatography on SiO₂ (hexane - 0% to 20% of ethyl acetate). 254.0 mg, 44% yield.
MS (ES): M/z= 217 (M+H)⁺
¹H NMR (400 MHz, DMSO-d₆, δ ppm): 9.53 (s, 1 H), 7.60 (d, J = 2.3 Hz, 1 H), 7.41 (s, 1 H), 7.35 (d, J = 8.0 Hz, 2H), 7.16 (d, J = 2.3 Hz, 1 H), 7.04 (d, J = 8.0 Hz, 1 H), 6.09 (s, 2H).

### Example 3. Synthesis of 5-(2-methoxy-pyridin-3-yl)-furan-2-carbaldehyde

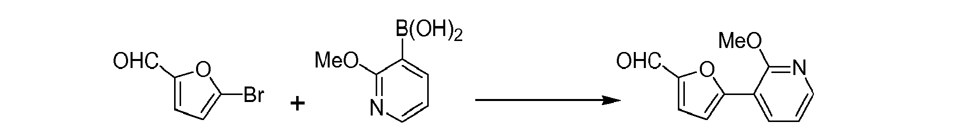

Synthesized using method A and purified by precipitation in diethyl ether/hexane (1:1). 352.0 mg, 64% yield.
MS (ES): M/z= 204 (M+H)⁺
¹H NMR (400 MHz, CDCl₃, δ ppm): 9.65 (s, 1 H), 8.29 (dd, J = 7.6, 1.8 Hz, 1 H), 8.19 (dd, J = 4.9, 1. 8 Hz, 1 H), 7.33 (d, J = 3.7 Hz, 1 H), 7.18 (d, J = 3.7 Hz, 1 H), 7.02 (dd, J = 7.6, 4.9 Hz, 1 H), 4.11 (s,3H).

### Example 4. Synthesis of 5-(2,4-dimethoxy-pyrimidin-5-yl)-furan-2-carbaldehyde

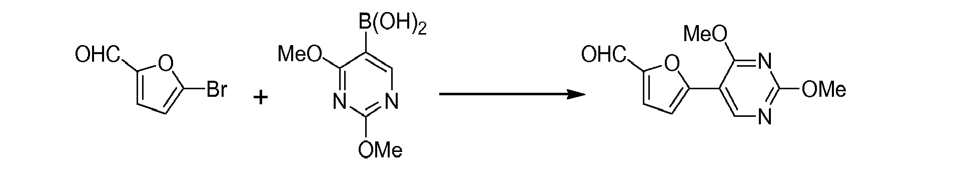

Synthesized using method A and purified by flash chromatography on SiO₂ (hexane - 0% to 20% of ethyl acetate). 472.0 mg, 75% yield.
MS (ES): M/z= 235 (M+H)⁺
¹H NMR (400 MHz, CDCl₃, δ ppm): 9.61 (s, 1 H), 8.86 (s, 1 H), 7.26 (d, J = 3.8 Hz, 1 H), 6.94 (d, J = 3.8 Hz, 1 H), 4.11 (s, 3H), 4.03 (s, 3H).

### Example 5. Synthesis of 5-(3,5-dimethyl-isoxazol-4-yl)-furan-2-carbaldehyde

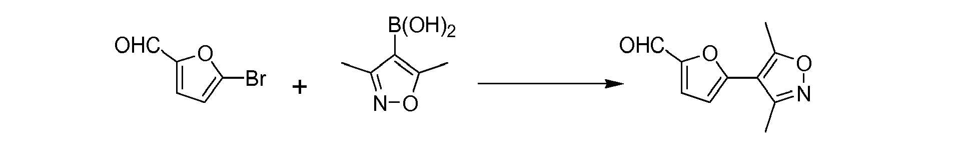

Synthesized using method A and purified by precipitation in diethyl ether. 325.0 mg, 63% yield.
MS (ES): M/z= 192 (M+H)⁺
¹H NMR (400 MHz, CDCl₃, δ ppm): 9.66 (s, 1 H), 8.86 (s, 1 H), 7.33 (d, 1 H, J = 3.8 Hz), 6.58 (d, J = 3.8 Hz, 1 H), 2.70 (s, 3H), 2.49 (s, 3H).

### Example 6. Synthesis of 5-pyrimidin-2-yl-furan-2-carbaldehyde

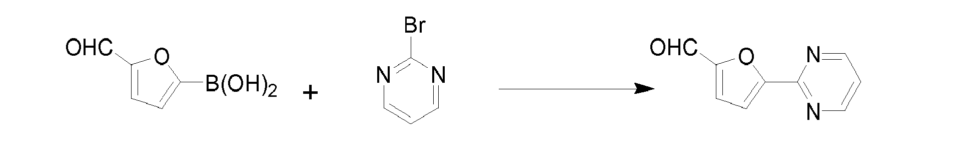

Synthesized using method B and purified by flash chromatography on SiO₂ (hexane - 0% to 30% of ethyl acetate). 105.0 mg, 22% yield.
MS (ES): M/z= 175 (M+H)⁺
¹H NMR (400 MHz, DMSO-d₆, δ ppm): 9.74 (s, 1H), 8.94 (d, J = 4.63 Hz, 2H), 7.69 (d, J=3.3Hz, 1H),7.53(m,2H).

### Example 7. Synthesis of 3-(5-formyl-furan-2-yl)-4-methoxy-benzoic acid methyl ester

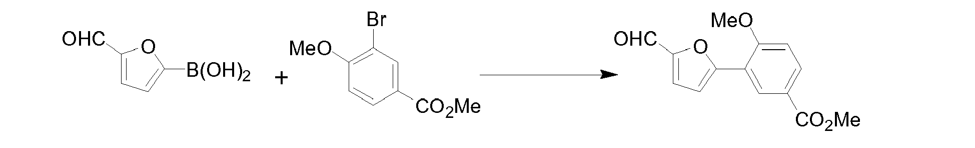

Synthesized using method B and purified by flash chromatography on SiO₂ (hexane - 0% to 30% of ethyl acetate). 278.0 mg, 39% yield.
MS (ES): M/z= 261 (M+H)⁺
¹H NMR (400 MHz, CDCl₃, δ ppm): 9.68 (s, 1 H), 8.66 (d, J = 2.3 Hz, 1 H), 8.05 (dd, J = 8.7, 2.3 Hz, 1H),7.31 (d, J = 3.6 Hz, 1 H), 7.12 (d, J = 3.6 Hz, 1 H), 7.02 (d, J = 8.7 Hz, 1 H), 3.99 (s, 3H), 3.91 (s, 3H).

### Condensation reactions of 2-formylfuranes with glycociamidine or isocreatinine

### Method C

A mixture of the selected aldehyde (1.0 eq.) and the imino-imidazolidinone compound glycociamidine [J. Am. Chem. Soc. 1953, 75, 3138-3140] or isocreatinine [J. Am. Chem. Soc. 1971, 93, 5552-5560] (1.2 eq.) in ethylene glycol (3.0 mL/100.0 mg imidazolidinone), was heated placed in a CEM Discover Labmate microwave oven at 160°C for 5 min. The solvent was evaporated till dryness in a speed-vac at 80°C for 16 hours. The final product was isolated from the crude by flash chromatography on SiO₂.

### Method D

A mixture of acetic acid (3.0 mL/100.0 mg imidazolone), sodium acetate (2.0 eq.), the selected aldehyde (1.0 eq.), and the imino-imidazolidinone compound (1.5 eq.) was heated at 110°C for 16 hours. The acid was evaporated and the resulting crude was treated with an organic solvent to precipitate the final product. Whenever there was no precipitation or the product was not pure, the crude was extracted in 20% methanol in methylene chloride (2 x 100 mL) and washed with water (2x30 mL). The combined organic layers were dried over anhydrous sodium sulfate, filtered and evaporated till dryness. The final product was isolated either by precipitation in ethyl ether or by flash chromatography on SiO₂.

### Example 8. Synthesis of -2-amino-5-[5-(4-nitro-phenyl)-furan-2-ylmethylene]-3,5 dihydro-imidazol-4-one (Compound 1).

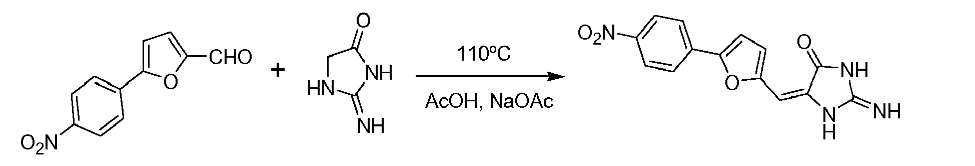

Synthesized following method D from glycociamidine (137.0 mg, 1.4 mmol), commercially available 5-(4-nitro-phenyl)-furan-2-carbaldehyde (250.0 mg, 1.1 mmol) and sodium acetate (187.0 mg, 2.3 mmoles). The final compound was isolated by precipitation in methanol and the solid was washed with diethyl ether to afford the pure imidazolone derivative as an orange solid. 62.0 mg, 18% yield.
MS (ES): M/z= 299 (M+H)⁺
¹H NMR (400 MHz, DMSO-d₆, δ ppm): 10.59 (s, 1H) 9.44 (s, 1H)8.54(s, 1H)8.29(d, J=8.5 Hz, 1H) 7.67 (d, J= 8.5 Hz, 1H), 7.53 (s, 1H), 7.41 (m, 1H) 7.18 (dd, J= 7.2, 1.7 Hz, 1H) 6.49 (s, 1H)

### Example 9. Synthesis of 2-amino-S-[S-(3-chloro-4-methoxy-phenyl)-furan-2-ylmethylene]-3-methyl-3,5-dihydro-imidazol-4-one (Compound 2).

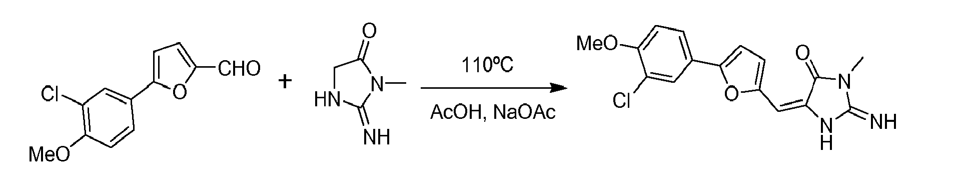

Synthesis was performed according to method D from isocreatinine (150.0 mg, 1.3 mmol), commercially available 5-(3-chloro-4-methoxy-phenyl)furfural (208.0 mg, 0.9 mmol), sodium acetate (144.0 mg, 1.8 mmol) and acetic acid (4.0 mL). The crude was extracted with 20% methanol in methylene chloride, and the final product was isolated by flash chromatography (SiO₂ 0-20% methanol in methylene chloride) as a yellow solid. 140.0 mg, 48% yield.
MS (ES): M/z= 332 (M+H)⁺
¹H NMR (400 MHz, DMSO-d₆, δ ppm): 7.78 (d, J= 2.0 Hz, 1 H, 2-H phenyl), 7.67 (dd, J= 8.7, 2.0 Hz, 1 H, 6-H phenyl), 7.63 (br.s, 2H, NH₂), 7.21 (d, J= 8.7 Hz, 1 H, 5-H phenyl), 7.12 (d, J= 3.5 Hz, 1 H, 3-H furanyl), 7.05 (d, J= 3.5 Hz, 1 H, 4-H furanyl), 6.31 (s, 1 H, CH=), 3.89 (s, 3H, O-CH₃), 3.05 (s, 3H, N-CH₃)
¹³C NMR (100 MHz, DMSO-d₆, δ ppm): 168.79 (4-C imidazolone), 159.49 (2-C imidazolone), 153.83 (4-C phenyl), 151.54 (2-C furanyl), 151.06 (5-C furanyl), 139.24 (5-C imidazolone), 124.77 (2-C phenyl), 123.68 (6-C phenyl), 123.44 (1-C phenyl), 121.64 (3-C phenyl), 114.66 (3-C furanyl), 113.20 (5-C phenyl), 108.45 (4-C furanyl), 99.86 (CH=), 56.15 (O-CH₃), 25.60 (N-CH₃)

### Example 10. Synthesis of 2-amino-5-[5-(2,4-dimethoxy-phenyl)-furan-2-ylmethylene]-3,5-dihydro-imidazol-4-one (Compound 3).

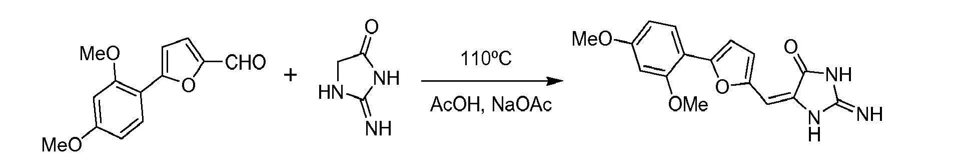

Synthesized according to method D from glycociamidine (100.0 mg, 1.0 mmol), 5-(2,4-dimethoxy-phenyl)-furan-2-carbaldehyde (195.0 mg, 0.8 mmol) and sodium acetate (137.0 mg, 1.7 mmol). The final compound was isolated by precipitation in water to yield the desired imidazolone as a brown solid. 229.0 mg, 87% yield.
MS (ES): M/z= 314 (M+H)⁺
¹H NMR (400 MHz, DMSO-d₆, δ ppm): 10.50 (br.s, 1H), 7.83 (m, 1H), 7.50 (br.s, 2H), 6.91 (s, 2H), 6.68 (m, 2H), 6.22 (s, 1 H), 3.92 (s, 3H), 3.82 (s, 3H).
¹³C NMR (100 MHz, DMSO-d₆, δ ppm): 172.08, 160.18, 156.63, 150.39, 148.95, 126.59, 114.71, 111.62, 110.95, 105.53, 98.60, 96.97, 55.58, 55.32.

### Example 11. Synthesis of 2-amino-5-(5-benzo[1,3]dioxol-5-yl-furan-2-ylmethylene)-3,5-dihydro-imidazol-4-one (Compound 4).

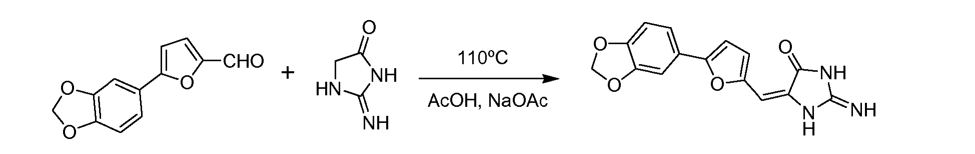

Synthesized following method D from glycociamidine (100.0 mg, 1.0 mmol), 5-benzo [1,3]dioxol-5-yl-furan-2-carbaldehyde (182.0 mg, 0.8 mmol) and sodium acetate (137.0 mg, 1.7 mmol). The final compound was isolated by precipitation in water as a brown solid. 229.0 mg, 87% yield.
MS (ES): M/z= 298 (M+H)⁺
¹H NMR (400 MHz, DMSO-d₆, δ ppm): 10.00 (br.s, 1 H), 7.42 (s, 1 H), 7.36 (s, 1 H), 7.20 (br.s, 2H), 6.99 (d, 2H, J = 8.97 Hz), 6.89 (s, 1 H), 6.20 (s, 1 H), 6.07 (s, 2H).

### Example 12. Synthesis of 2-amino-5-[5-(2-methoxy-pyridin-3-yl)-furan-2-ylmethylene]-3,5-dihydro-imidazol-4-one (Compound 5).

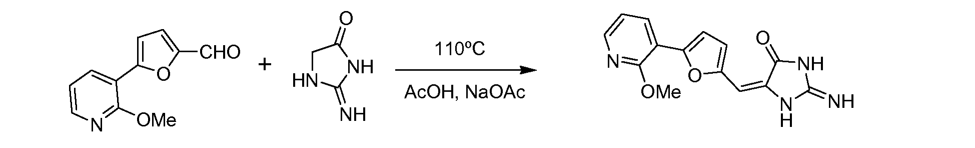

Synthesis was accomplished following method D from glycociamidine (100.0 mg, 1.0 mmol) 5-(2-methoxy-pyridin-3-yl)-furan-2-carbaldehyde (171.0 mg, 0.8 mmol) and sodium acetate (137.0 mg, 1.7 mmol). The crude was extracted with 20% methanol in methylene chloride and, after evaporation of the organic layer the resulting oil was precipitated in ethyl ether affording the pure imidizalone derivative as a yellow solid. 48.0 mg. 20% yield.
MS (ES): M/z= 285 (M+H)⁺
¹H NMR (400 MHz, DMSO-d₆, δ ppm): 10.40 (br.s, 1 H), 8.27 (s, 1 H), 8.13 (m, 1 H), 7.4 (br.s, 2H), 7.13 (m, 2H), 6.98 (s, 1 H), 6.25 (s, 1 H), 4.02 (s, 3H).
¹³C NMR (100 MHz, DMSO-d₆, δ ppm): 171.96, 158.33, 150.93, 148.07, 145.06, 133.52, 117.33, 117.32, 117.27, 114.48, 113.65, 113.06, 53.49.

### Example 13. Synthesis of 2-amino-5-[5-(2,4-dimethoxy-pyrimidin-5-yl)-furan-2-ylmethylene]-3,5-dihydro-imidazol-4-one (Compound 6).

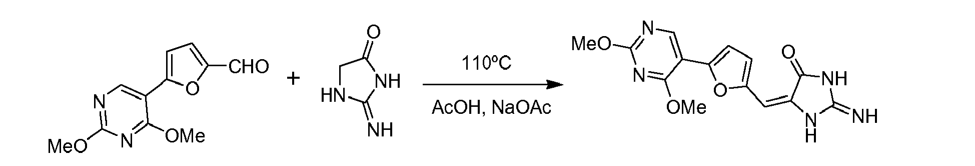

Syntesis was performed according to method D from glycociamidine (100.0 mg, 1.0 mmol), 5-(2, 4-dimethoxy-pyrimidin-5-yl)-furan-2-carbaldehyde (197.0 mg, 0.8 mmol) and sodium acetate (137.0 mg, 1.7 mmol). The final compound was isolated by precipitation in water as a brown solid. 143.0 mg, 60%yield.
¹H NMR (400 MHz, DMSO-d₆, δ ppm): 9.20 (s, 1 H), 7.04 (d, J = 3.2 Hz, 1 H), 6.93 (d, J = 3.2 Hz, 1 H), 6.09 (s, 1 H), 4.06 (s, 3H), 3.94 (s, 3H), 3.40 (br.s, 3H).
¹³C NMR (100 MHz, DMSO-d₆, δ ppm): 173.06, 167.41, 166.06, 163.62, 155.02, 150.32, 146.98, 130.74, 114.70, 112.74, 106.41, 95.94, 55.05, 54.69.

### Example 14. Synthesis of 2-amino-5-[5-(3,5-dimethyl-isoxazol-4-yl)-furan-2-ylmethylene]-3-methyl-3,5-dihydro-imidazol-4-one (Compound 16).

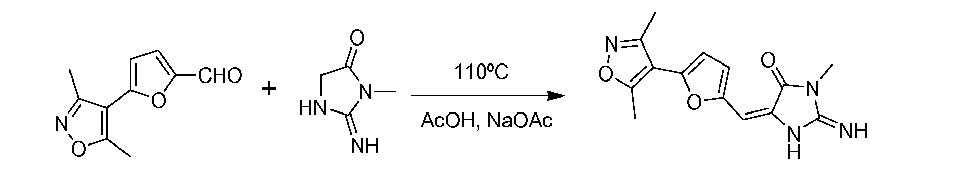

Synthesis was performed as described in method D from isocreatinine (100.0 mg, 1.2 mmol), 5-(3,5-dimethyl-isoxazol-4-yl)-furan-2-carbaldehyde (141.0 mg, 1.0 mmol) and sodium acetate (121.0 mg, 1.5 mmol). The crude was extracted with 20% methanol in methylene chloride and, after evaporation of the organic layer the resulting oil was precipitated in ethyl ether affording the pure imidizalone derivative as a yellow solid. 138.0 mg, 65% yield.
¹H NMR (400 MHz, DMSO-d₆, δ ppm): 7.60 (br.s, 2H), 7.10 (d, J = 3.2 Hz, 1 H), 6.74 (d, J = 3.20 Hz, 1 H), 6.31 (s, 1 H), 3.04 (s, 3H), 2.63 (s, 3H), 2.39 (s, 3H).
¹³C NMR (100 MHz, DMSO-d₆, δ ppm): 168.78, 165.51, 159.47, 157.10, 151.14, 144.22, 139.06, 113.74, 109.80, 107.47, 99.75, 25.59, 12.21, 11.19.

### Example 15. Synthesis of 2-amino-5-(5-pyrimidin-2-yl-furan-2-ylmethylene)-3,5-dihydro-imidazol-4-one (Compound 8).

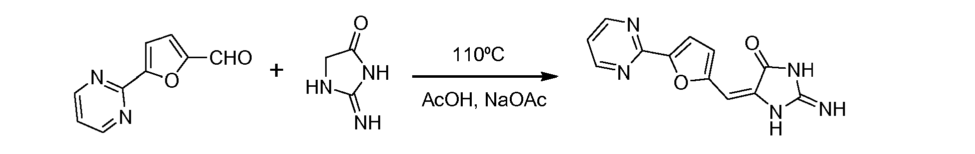

Synthesized according to method D from glycociamidine (72.0 mg, 0.7 mmol), 5-pyrimidin-2-yl-furan-2-carbaldehyde (105.0 mg, 0.6 mmol) and sodium acetate (98.0 mg, 1.2 mmol0). The crude was extracted with 20% methanol in mehylene chloride and, after evaporation of the organic layer the resulting oil was precipitated in ethyl ether affording the pure imidizalone derivative as a brown solid. 67.0 mg, 44% yield.
¹H NMR (400 MHz, DMSO-d₆, δ ppm): 10.20 (br.s. 1 H), 8.82 (d, J = 4.3 Hz, 2H), 7.60 (br s, 2H), 7.43 (d, J = 3.0 Hz, 1 H), 7.34 (t, J = 4.3 Hz, 1 H), 7.12 (s, 1 H), 6.20 (s, 1 H). ¹³C NMR (100 MHz, DMSO-d₆, δ ppm): 172.77, 158.60, 158.27, 157.25, 154.65, 153.74, 151.59, 119.82, 116.88, 115.50, 114.83, 114.59.

### Example 16. Synthesis of 3-[5-(2-amino-5-oxo-1,5-dihydro-imidazol-4-ylidenemethyl)-furan-2-yl]-4-methoxy-benzoic acid methyl ester (Compound 9).

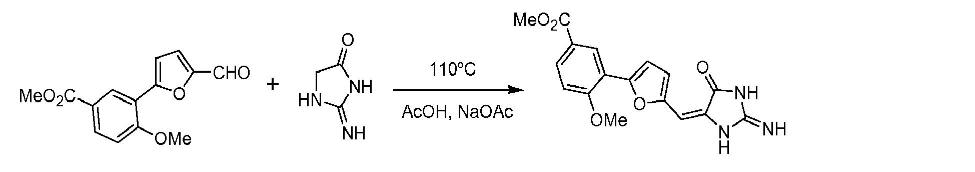

Synthesis was performed according to method D from glycociamidine (100.0 mg, 1.0 mmol0), 3-(5-formyl-furan-2-yl)-4-methoxy-benzoic acid methyl ester (191.0 mg, 0.8 mmol) and sodium acetate (137.0 mg, 1.7 mmol). The crude was extracted with 20% methanol in methylene chloride and, after evaporation of the organic layer the resulting oil was precipitated in ethyl ether affording the pure imidizalone derivative as a yellow solid. 86.0 mg, 30% yield.
¹H NMR (400 MHz, DMSO-d₆, δ ppm): 10.50 (br.s, 1 H), 8.35 (s, 1 H), 7.88 (d, J = 8.3 Hz, 1 H), 7.24 (d, J = 8.3 Hz, 2H), 7.20 (br.s, 2H), 7.11 (d, J = 3.1 Hz, 1 H), 6.23 (s, 1 H), 4.00 (s, 3H), 3.85 (s, 3H).
¹³C NMR (100 MHz, DMSO-d₆, δ ppm): 171.98, 165.75, 158.49, 150.81, 148.01, 131.97, 129.84, 126.19, 122.03, 118.50, 114.40, 113.90, 113.23, 112.30, 111.72, 56.07, 51.95.

### Spectral Data

The following compounds of formula (I) have also been prepared following the general methods described herein.

### Example 17: Spectral data of 2-Amino-5-[5-(3,5-dimethyl-isoxazol-4-yl)-furan-2-ylmethylene]-3,5-dihydro-imidazol-4-one (Compound 7)

**1 H-NMR (DMSO-d₆, 400 MHz, ppm):** 6.97 (s, 1 H), 6.76 (d, 1 H, J=3.5Hz) , 6.22 (s, 1 H), 2.60 (s, 3H), 2.38 (s, 3H).

**13C-NMR (DMSO-d₆, 100 MHz, ppm):** 172.7,166.6,158.1, 151.3, 145.3, 114.1, 110.9, 108.3, 97.7, 97.6, 97.6, 12.9, 11.8.

### Example 18: Spectral data of 2-Amino-5-[5-(3-chloro-4-methoxy-phenyl)-furan-2-ylmethylene]-3,5-dihydro-imidazol-4-one (Compound 10)

**1 H-NMR (DMSO-d₆, 400 MHz, ppm):** 7.79 (m, 2H), 7.21 (m, 2H), 7.06 (s, 1 H), 6.22 (s, 1 H), 3.87 (s, 3H).

### Example 19: Spectral data of 3-[5-(2-Amino-1-methyl-5-oxo-1,5-dihydro-imidazol-4-ylidenemethyl)-furan-2-yl]-4-methoxy-benzonitrile (Compound 11) 1H-NMR (DMSO-d₆, 400 MHz, ppm): 8.13 (d, 1 H, J=2.1 Hz), 7.75 (dd, 1 H, J=2.1, 8.6 Hz), 7.30 (d, 1 H, J=8.7 Hz), 7.16 (m, 2H), 6.36 (s, 1 H), 4.02 (s, 3H),, 3.05 (s, 3H). 13C-NMR (DMSO-d₆, 100 MHz, ppm): 168.7, 159.7, 157.9, 151.6, 146.7, 139.7, 132.4, 128.4,119.5,118.8,114.7,114.6,112.7,103.3,99.5,56.2,25.6.

### Example 20: Spectral data of 2-Amino-3-benzyl-5-[5-(3-chloro-4-methoxy-phenyl)-furan-2-ylmethylene]-3,5-dihydro-imidazol-4-one (Compound12)

**1 H-NMR (DMSO-d₆, 400 MHz, ppm):** 7.78 (m, 1 H), 7.67 (m, 3H), 7.34 (m, 2H), 7.25 (m, 4H), 7.13 (m, 1 H), 7.06 (m, 1 H), 6.35 (s, 1H),, 4.80 (m, 2H),, 3.88(m, 3H).

**13C-NMR (DMSO-d₆, 100 MHz, ppm):** 168.8, 158.6, 153.9, 151.5, 151.3, 138.6, 136.6, 128.4, 127.3, 126.9, 124.8, 123.6, 123.5, 121.7, 115.0, 113.2, 108.5, 100.4, 56.2, 41.9.

### Example 21: Spectral data of 2-Amino-5-[5-(3-chloro-4-methoxy-phenyl)-furan-2-ylmethylene]-3-(2-methoxy-ethyl)-3,5-dihydro-imidazol-4-one (Compound 13)

**1 H-NMR (DMSO-d₆, 400 MHz, ppm):** 7.79 (d, 1 H, J=2.1 Hz), 7.68 (d, 1 H, J=2.1, 7.6 Hz), 7.56 (s, 2H), 7.22 (d, 1 H, J=8.8 Hz), 7.12 (d, 1 H, J=3.3 Hz), 7.06 (d, 1 H, J=3.5 Hz), 6.30 (s, 1 H), 3.89 (s, 3H), 3.74 (t, 2H, J=5.5 Hz), 3.46(t, 2H, J=5.5 Hz), 3.25 (s, 3H).

**13C-NMR (DMSO-d₆, 100 MHz, ppm):** 168.7,159.0,153.8,151.5,151.1,138.9, 124.8, 123.7, 123.5, 121.6, 114.7, 113.2, 108.5, 99.9, 69.1, 58.0, 56.2, 38.4.

### Example 22: Spectral data of 2-Amino-5-[5-(2-methoxy-pyridin-3-yl)-furan-2-ylmethylene]-3-methyl-3,5-dihydro-imidazol-4-one (Compound 14)

**1H-NMR (DMSO-d₆, 400 MHz, ppm):** 8.15 (dd, 1 H, J=1.8, 7.6 Hz), 8.12 (dd, 1 H, J=1.8, 4.8 Hz), 7.18 (d, 1 H, J=3.5 Hz), 7.14 (d, 1 H, J=3.8 Hz), 7.11 (dd, 1 H, J=4.9, 7.5 Hz), 6.35 (s, 1 H), 4.02 (s, 3H), 3.05 (s, 3H).

**13C-NMR (DMSO-d₆, 100 MHz, ppm):** 172.0, 168.4, 159.5, 158.3, 151.6, 147.5, 145.0, 133.2, 117.3, 114.9, 113.9, 113.2, 99.8, 53.5, 25.7.

### Example 23: Spectral data of 2-Amino-5-[5-(2,4-dimethoxy-phenyl)-furan-2-ylmethylene]-3-methyl-3,5-dihydro-imidazol-4-one (Compound 15)

**1 H-NMR (DMSO-d₆, 400 MHz, ppm):** 7.74 (d, 1 H, J=9.2Hz), 7.13 (d, 1 H, J=3.1 Hz), 6.91 (d, 1 H, J=3.2Hz), 6.67 (s, 1 H), 6.64 (d, 1 H, J=9.2Hz), 6.35 (s, 1 H), 3.91 (s, 3H), 3.81 (s, 3H), 3.05 (s, 3H).

**13C-NMR (DMSO-d₆, 100 MHz, ppm):** 171.9, 168.3, 160.1, 158.9, 156.5, 149.6, 137.2, 126.2, 115.3, 111.7, 111.2, 105.6, 100.5,98.6,55.5,55.3,25.6.

### Example 24: Spectral data of 2-Amino-5-[5-(4-methoxy-phenyl)-furan-2-ylmethylene]-3,5-dihydro-imidazol-4-one (Compound 17)

**1 H-NMR (DMSO-d₆, 400 MHz, ppm):** 7.76 (m, 2H), 6.97 (m, 4H), 6.21 (s, 1 H), 3.80 (s, 3H).

**13C-NMR (DMSO-d₆, 100 MHz, ppm):** 172.0, 159.0, 158.8, 153.6, 150.1, 149.6, 126.7,125.3,125.0,122.8,122.6,114.6,114.3,107.1, 55.2.

### Example 25: Spectral data of Acetate 1-methyl-4-[5-(4-nitro-phenyl)-furan-2-ylmethylene]-5-oxo-4,5-dihydro-1H-imidazol-2-yl-ammonium (Compound 18)

**1 H-NMR (DMSO-d₆, 400 MHz, ppm):** 8.26 (s, 1 H), 8.24 (s, 1 H), 7.95 (s, 1 H), 7.93 (s, 1 H), 7.76 (s ancho, 3H), 7.42 (dd, 1 H, J=0.5Hz, J=3.6Hz), 7.20 (d, 1 H, J=3.6Hz), 6.30 (s, 1 H), 3.04 (s, 3H) ,1.89 (s, 3H).

### Biological Methods

### Example 26: BACE assay

This assay is based on time-resolved fluorescence ("TRF") technology, using a. BACE peptide substrate containing a fluorescent europium chelate coupled to one end and a quencher coupled to the other end. BACE cleaves the peptide separating the europium chelate and the quencher, hence increasing the fluorescence of the Eu chelate. The reagents which are used in this assay are BACE-1 β-Secretase recombinant human enzyme (R&D Systems, Minneapolis, MN), TruPoint™ BACE1 substrate (Perkin Elmer Waltham, MA), sodium acetate, CHAPS and EDTA (Sigma Aldrich, St Louis, MO). The assay is carried out in a 96-well microplate in a final volume of 100 µl using 200 nM peptide substrate and 8 nM enzyme in 50 mM sodium acetate pH 4.5 with 1 mM EDTA and 0.2% (w/v) CHAPS. The plates are incubated for 1 h in the dark at room temperature and then read in a fluorimeter plate reader provided with time-resolved fluorescence capabilities (PolarStar Optima™, BMG-Labtech, Offenburg, Germany) using 337 nm as excitation wavelength and 615 nm as the emission wavelength. Compounds are tested in duplicate using a concentration range extending from 5 nM to 100 µM, using 10 µM H-4848 (commercial BACE inhibitor, BACHEM, Bubendorf, Switzerland) as control.

The compounds of formula (I) of the present invention where submitted to the above indicated assay, in order to determine their BACE activity inhibition. The results, indicated in IC₅₀ format, are indicated below in **Table I.** IC₅₀ is the half maximal inhibitory concentration, which is a measure of the effectiveness of a compound in inhibiting biological or biochemical function. This quantitative measure indicates the concentration of a particular substance is needed to inhibit a given biological process by half. In other words, it is the half maximal (50%) inhibitory concentration (IC) of a substance.

### Example 27: Inhibition of beta-amyloid secretion

To quantify human Aβ1-40 an ELISA-based commercial kit was used (Immunoassay Kit Human β Amyloid 1-40, The Genetics Company, Schlieren, Switzerland). Aβ (1-40) peptide was quantified from cell culture supernatants using a CHO7w cell line stably transfected with human BACE. β-Secretase inhibitor IV (Calbiochem, San Diego, CA), was used as Aβ secretion reduction positive control in all the Aβ secretion studies. Cells were grown in DMEM supplemented with 2% Foetal bovine serum, 1% penicillin-streptomycin, 1% L-glutamine and 200 µg/ml G418. Cells were seeded in 96-well culture microplates at 5000 cells/well and treated with 10 µM β secretase inhibitor IV or compounds for 48 hours. Then cell culture supernatants were collected and analyzed according to the kit manufacturer's instructions. At 10 µM β-secretase inhibitor IV the inhibition of Aβ secretion inhibition is usually between 70 and 100 %.

Some of the compounds of formula (I) of the present invention where submitted to the above indicated assay, in order to determine their capacity of inhibiting Aβ secretion. The results, indicated in IC₅₀ format, are indicated below in **Table I.**

**Table I**

| | **IC50 Inhibition of BACE Activity (**µ**M)** | **IC50 Inhibition of A**β **secretion** (µ**M**) | |
|---|---|---|---|
| **Compound 1** | 0.11 | / | 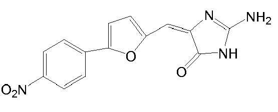 |
| **Compound 2** | 0.326 | / | 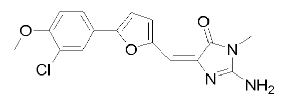 |
| **Compound 3** | 0.369 | / | 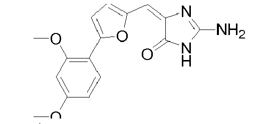 |
| **Compound 4** | 0.25 | 30 | 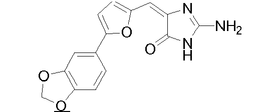 |
| **Compound 5** | 0.36 | 40 | 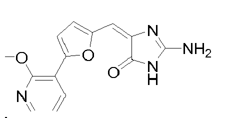 |
| **Compound 6** | 1.32 | / | 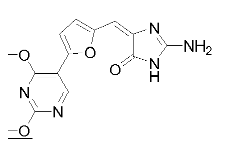 |
| **Compound 7** | 0.39 | / | 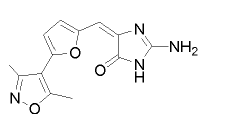 |
| **Compound 8** | 1.3 | / | 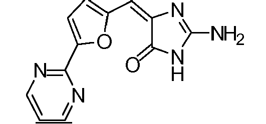 |
| **Compound 9** | 0.711 | 45 | 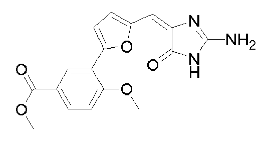 |
| **Compound 10** | 0.319 | / | 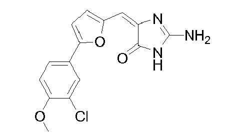 |
| **Compound 11** | 0.22 | / | 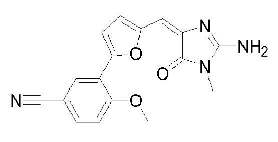 |
| **Compound 12** | 1.23 | / | 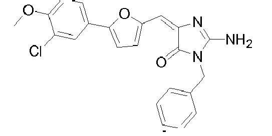 |
| **Compound 13** | 0.81 | / | 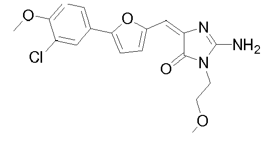 |
| **Compound 14** | 0.54 | / | 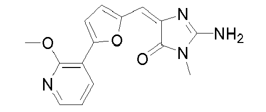 |
| **Compound 15** | 0.346 | / | 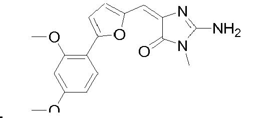 |
| **Compound 16** | 1.23 | / | 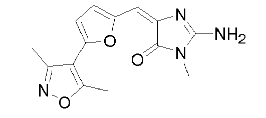 |
| **Compound 17** | 0.422 | 35 | 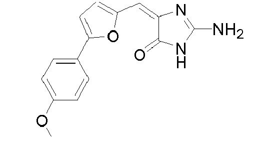 |
| **Compound 18** | 0,4 | / | 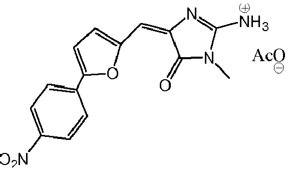 |

| | | | |
|---|---|---|---|
| "/" = not assayed | | | |

## Claims

1. A compound of formula (I), or a tautomer, salt, solvate or prodrug thereof 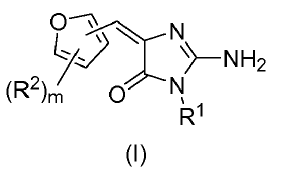 wherein
R¹ is selected from hydrogen, substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl and substituted or unsubstituted heteroaryl;
each R² is independently selected from hydrogen, substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl, substituted or unsubstituted heteroaryl, trifluoromethyl, cyano, -N(Rₐ)(R_{b}), -ORₐ, -SRₐ, -C(O)Rₐ, -C(O)ORₐ and -C(O)N(Rₐ)(R_{b}), wherein each Rₐ and R_{b} are independently selected from hydrogen and alkyl, substituted or unsubstituted alkenyl, and substituted or unsubstituted alkynyl;
m is an integer selected from 1, 2 and 3;
with the proviso that:
- 2-amino-5-(furan-2-ylmethylene)-1,5-dihydro-imidazol-4-one, and
- 2-amino-3-methyl-5-(5-methyl-furan-2-ylmethylene)-3,5-dihydro-imidazol-4-one
are not included in formula (I).

2. A compound according to claim 1, wherein R¹ is selected from hydrogen, substituted or unsubstituted C₁₋₃ alkyl, substituted or unsubstituted arylmethyl, substituted or unsubstituted heteroarylmethyl, substituted or unsubstituted phenyl, and substituted or unsubstituted heteroaryl.

3. A compound according to claim 2, wherein R¹ is selected from hydrogen, methyl, substituted or unsubstituted benzyl, substituted or unsubstituted heteroarylmethyl, substituted or unsubstituted phenyl and methyloxyethyl.

4. A compound according to any previous claim, of general formula (Ia) 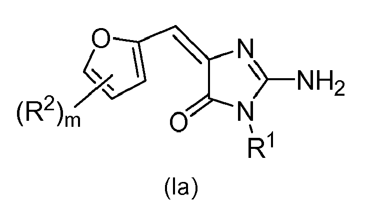 wherein R¹, R² and m are as defined in claim 1,
or a tautomer, salt, solvate or prodrug thereof.

5. A compound according to any previous claim, wherein m is 1 or 2.

6. A compound according to any previous claim, of general formula (Ib) 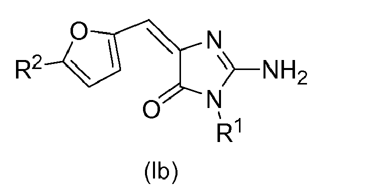 wherein R¹ and R² are as defined in claim 1,
or a tautomer, salt, solvate or prodrug thereof.

7. A compound according to any previous claim, wherein R² is selected from hydrogen, C₁₋₃ alkyl, substituted or unsubstituted phenyl and substituted or unsubstituted 5- or 6-membered heteroaryl ring.

8. A compound according to any previous claim, wherein R² is hydrogen or a radical selected from methyl, phenyl, pyridine, pyrimidine or isoxazol optionally substituted by methyl, methoxy, methoxycarbonyl, chloro, nitro, cyano or [1,3]dioxolane.

9. A compound according to any previous claim, wherein R² is selected from substituted or unsubstituted aryl and substituted or unsubstituted heteroaryl.

10. A compound according to claim 1, selected from: 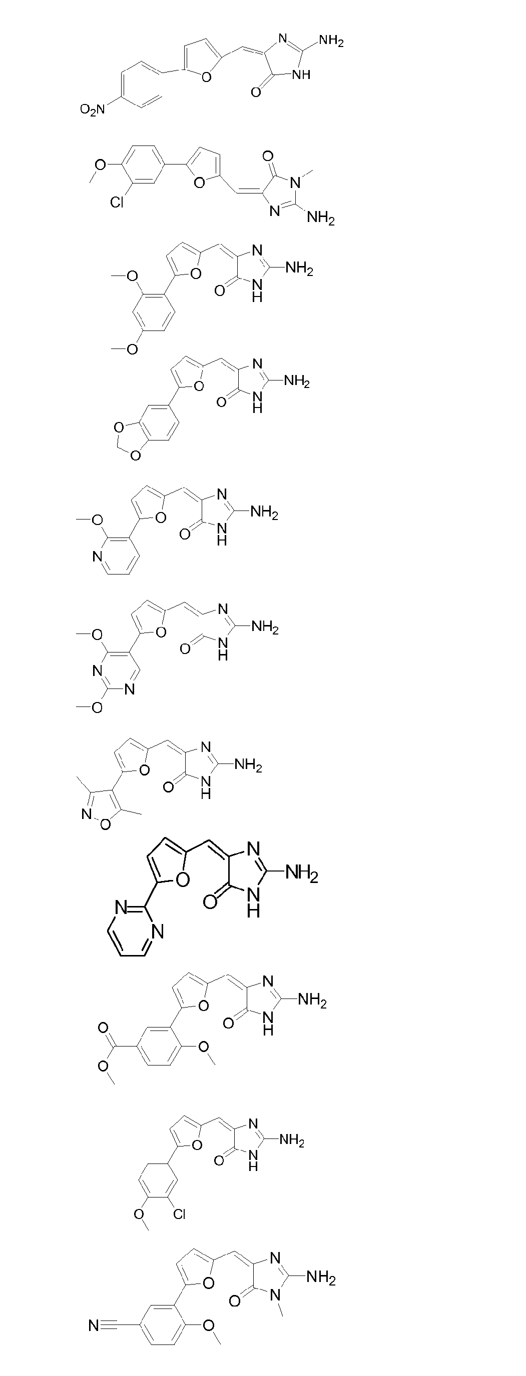 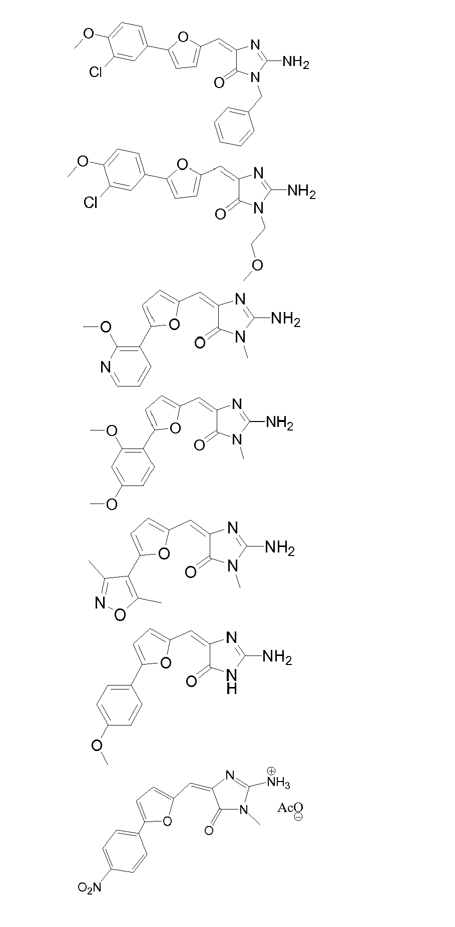 or a tautomer, salt, solvate or prodrug thereof.

11. A pharmaceutical composition comprising at least one compound of formula (I), or a tautomer, salt, solvate or prodrug thereof, 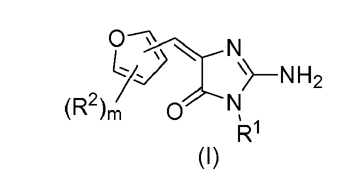 wherein
R¹ is selected from hydrogen, substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl and substituted or unsubstituted heteroaryl;
each R² is selected from hydrogen, substituted or unsubstituted alkyl, substituted or unsubstituted alkenyl, substituted or unsubstituted alkynyl, substituted or unsubstituted arylalkyl, substituted or unsubstituted heteroarylalkyl, substituted or unsubstituted aryl, substituted or unsubstituted heteroaryl, trifluoromethyl, cyano, - N(Rₐ)(R_{b}), -ORₐ, -SRₐ, -C(O)Rₐ, -C(O)ORₐ and -C(O)N(Rₐ)(R_{b}), wherein each Rₐ and R_{b} are independently selected from hydrogen and alkyl, substituted or unsubstituted alkenyl, and substituted or unsubstituted alkynyl;
m is an integer selected from 1, 2 and 3;
and at least one pharmaceutically acceptable carrier, adjuvant and/or vehicle.

12. A compound of formula (I), or a tautomer, salt, solvate or prodrug thereof, as defined in claim 11, for use as a medicament.

13. A compound of formula (I), or a tautomer, salt, solvate or prodrug thereof, as defined in claim 11, for use as a medicament for the treatment and/or prophylaxis of a cognitive, neurodegenerative or neuronal disease or disorder.

14. A compound according to claim 13, wherein the cognitive, neurodegenerative or neuronal disease or disorder is selected from chronic neurodegenerative conditions including dementias such as Alzheimer's disease, memory loss, attention deficit symptoms associated with Alzheimer's disease, diffuse Lewy body type Alzheimer's disease, mild cognitive impairment, Hereditary Cerebral Haemorrhage with Amyloidosis of the Dutch-Type, β-amyloid angiopathy and cerebral bleeding such as cerebral bleeding due to solitary cerebral amyloid angiopathy, prion infections, degenerative dementias, including dementias of mixed vascular and degenerative origin, frontotemporal dementia, pre-senile dementia, senile dementia, AIDS associated dementia, parkinsonian disorders such as Parkinson's disease (PD), subacute sclerosing panencephalitic parkinsonism, postencephalitic parkinsonism, pugilistic encephalitis, guam parkinsonism-dementia complex, Pick's disease, multiple system atrophy (MSA), progressive supranuclear palsy (PSP), and corticobasal degeneration (CBD), Down syndrome, Lewy body disease, Huntington's Disease, amyotrophic lateral sclerosis, multiple sclerosis and neurotraumatic diseases such as acute stroke, epilepsy, promotion of functional recovery post stroke, ischaemia, brain injury, especially traumatic brain injury and neuroinflammation.

15. Use of a compound according to formula (I), or a tautomer, salt, solvate or prodrug thereof, as defined in claim 11, as a reactive in an in vitro biological assay for inhibiting BACE and beta-amyloid secretion.
